# EUROPEAN PATENT SPECIFICATION

(11) **EP 1 802 771 B1**
(45) Date of publication and mention of the grant of the patent: **15.08.2012**
(21) Application number: 05797166.5
(22) Date of filing: 29.09.2005
(51) Int. Cl.: C12Q 1/68

(54) **DETECTION, IDENTIFICATION AND DIFFERENTIATION OF SERRATIA SPECIES USING THE SPACER REGION**
NACHWEIS, IDENTIFIZIERUNG UND DIFFERENZIERUNG VON SERRATIA-SPEZIES UNTER VERWENDUNG DES SPACER-BEREICHS
DETECTION, IDENTIFICATION ET DIFFERENTIATION DE SERRATIA SPECIES UTILISANT LA REGION INTERGENIQUE

(30) Priority: 30.09.2004 EP 04447218; 08.12.2004 US 634106 P
(43) Date of publication of application: 04.07.2007
(73) Proprietor: Innogenetics N.V., 9052 Ghent (BE); Roche Diagnostics GmbH, 68305 Mannheim (DE)
(72) Inventor: JANNES, Geert, B-3010 Leuven (BE); MIJS, Wouter, B-9820 Merelbeke (BE); EMRICH, Thomas, 82393 Iffeldorf (DE); HABERHAUSEN, Gerd, 82377 Penzberg (DE)
(74) Representative: BiiP cvba
(86) International application number: PCT/EP2005/054913
(87) International publication number: WO 2006/035062

(56) References cited:
- EP-A- 1 091 004
- WO-A-91/16454
- WO-A-2005/075673
- KUR ET AL.: "Identificaton of Serratia marcescens on the basis of polymerase chain reaction-amplified ribosomal DNA spacer polymorphisms" ACTA MICROBIOL. POL., vol. 44, no. 3-4, 1995, pages 219-225, XP008044336
- DATABASE EMBL 18 August 2003 (2003-08-18), PAVCO ET AL: "Method and reagent for the treatment of diseases or conditions related to the levels of vascular endothelial growth factor receptor" XP002360438 retrieved from EBI Database accession no. AR323752
- DATABASE EMBL 16 January 2004 (2004-01-16), DONNER ET AL: "Biochip" XP002360439 retrieved from EBI Database accession no. AX989045
- IWAYA AKIRA ET AL: "Nosocomial outbreak-derived Serratia marcescens: Rapid and quantitative detection of the agent in the blood by a real time PCR assay." ACTA MEDICA ET BIOLOGICA, vol. 50, no. 4, December 2002 (2002-12), pages 185-190, XP008043080 ISSN: 0567-7734 & DATABASE GENEBANK 17 November 1999 (1999-11-17), SPROEER: "Serratia marcescens 16SrRNA (strain DSM 30121)" Database accession no. AJ233431
- PATTON TONI G ET AL: "Genotyping of clinical Serratia marcescens isolates: A comparison of PCR-based methods" FEMS MICROBIOLOGY LETTERS, vol. 194, no. 1, 1 January 2001 (2001-01-01), pages 19-25, XP002320860 ISSN: 0378-1097
- DATABASE GENBANK SCHABEL E.L. ET AL: 'Erwinia amylovora strain Ea110 16S-23S ribosomal RNA intergenic spacer, tRNA-Ile and tRNA-Ala genes, complete sequences.' Database accession no. AF449660
- GRIMONT ET AL: "Ribosomal ribonucleic acid gene restriction patterns as potential taxonomic tools", ANNALES DE L'INSTITUT PASTEUR / MICROBIOLOGIE, PARIS, FR, vol. 137, no. 1, 8 July 1986 (1986-07-08), pages 165-175, XP022298104, ISSN: 0769-2609
- DAUGA C ET AL: "NUCLEOTIDE SEQUENCES OF 16S RIBOSOMAL RNA FROM TEN SERRATIA-SPP", RESEARCH IN MICROBIOLOGY, vol. 141, no. 9, 1990, pages 1139-1150, ISSN: 0923-2508

## Description

### FIELD OF THE INVENTION

The present disclosure relates to new nucleic acid sequences derived from the ITS (Internal Transcribed Spacer) region, between the 16S and 23S ribosomal ribonucleic acid (rRNA) genes, to be used for the specific detection and/or identification of *Serratia* species, in particular of *Serratia marcescens, Serratia ficaria,* and/or *Serratia fonticola.*

The present disclosure relates also to a method for the specific detection and/or identification of *Serratia* species, in particular *Serratia marcescens, Serratia ficaria,* and/or *Serratia fonticola* using new nucleic acid sequences derived from the ITS region.

### BACKGROUND OF THE INVENTION

The genus *Serratia,* of the family *Enterobacteriaceae,* consists of 11 species : *Serrtia entomophila, Serratia ficaria, Serratia fonticola, Serratia liquefaciens* group *(S. liquefaciens* s.s., *S*. *grimesii* and *S. proteamaculans), Serratia marcescens, Serratia odorifera, Serratia plymuthica, Serratia quinivorans* and *Serratia rubidaea.*

Members of the genus *Serratia* are found in water and soil and on leaves, fruits, vegetables, mushrooms, mosses and insects. The only *Serratia* species that has been routinely associated with human disease is *S*. *marcescens.* Most other species of *Serratia* have also been isolated from humans, where they are usually transiently present and can cause opportunistic infections. Only *S*. *entomophila* has not been isolated from humans so far.

The most common sites for *Serratia* infection include the urinary tract, the respiratory tract and the bloodstream. CNS infection mostly occurs following neurosurgery. *Serratia* is a virulent organism. When it enters the bloodstream endotoxins are released and cause fever, septic shock, thrombocytopaenia and disseminated intravascular coagulation. The mortality from *Serratia* bacteraemia is high. *Serratia* is naturally resistant to some antibiotics and can quickly become resistant to other antibiotics due to enzyme production.

Nosocomial transmission of *Serratia* most often occurs from contaminated hands of health care workers. Contaminated equipment is another source of *Serratia* infection. Urinary tract infections are almost always associated with indwelling catheters. Respiratory tract infections are common in patients residing in places which are being mechanically ventilated. If a person is colonised with *Serratia,* they easily become infected due to invasive devices, surgery and severity of illness.

Currently the *Serratia species* are identified and differentiated by culture based methods and phenotypic biochemical tests.

They grow well on enriched media (e.g. blood agar) at 35 to 37°C. They are non-lactose fermenters and are motile. *Serratia* species produce extracellular DNase at 25°C and gelatinase at 22°C. They elaborate lipase and are resistant to colistin, ampicillin and cephalothin. These properties are specific to *Serratia* and are not characteristic for any other *Enterobacteriaceae.* For many years, *S*. *marcescens* was differentiated from other enteric bacteria due to its characteristic red pigmentation. However, not all *S*. *marcescens* organisms produce the typical red pigment prodigiosin. The various *Serratia* species can be distinguished from *S*. *marcescens* by decarboxylation and fermentation reactions.

J. Kur et al. ((1995) Acta Microbiologica Polonica 44 (3/4): 219-225) describe the amplification of the rDNA 16S - 23S spacer region of *S*. *marcescens* organisms and fragmentation thereof by using a restriction nuclease. Nor the spacer regions neither the fragments thereof have been disclosed. Identification of *S*. *marcescens* from other *Serratia* species by application this method has not been demonstrated

From a health safety standpoint, *S*. *marcescens* is of great concern due to its increasing number of cases, virulence and its increasing resistance to antibiotics. A rapid and specific identification assay will therefore be the basis for a more appropriate antimicrobial management of infections caused by this organism.

### SUMMARY

It is an object of the present disclosure to provide new nucleic acid sequences derived from the ITS of *Serratia* species, which can be used, for the detection and/or identification of *Serratia* species, in particular of *Serratia marcescens, Serratia ficaria,* and/or *Serratia fonticola.*

An isolated nucleic acid molecule selected from the group consisting of SEQ ID NOs 1 to 57, their complementary form, the RNA form thereof wherein T is replaced by U, and homologues are thus provided.

The use of said nucleic acid molecules and of fragments thereof for the detection and/or identification of *Serratia* species is also an object of the present disclosure. It will be clear to the skilled in the art that the tRNA sequences encoding glutamine, isoleucine and alanine are not suitable for said detection and/or identification.

An aspect relates to new polynucleotides for use as probes and/or primers, for the detection and/or identification of *Serratia* species, in particular of *Serratia marcescens, Serratia ficaria,* and/or *Serratia fonticola.*

The present disclosure thus provides an isolated nucleic acid molecule that specifically hybridizes to a target sequence comprising or consisting of a nucleic acid molecule selected from the group consisting of SEQ ID NOs 12 to 57, the complementary form thereof, the RNA form thereof wherein T is replaced by U, homologous sequences thereof, and fragments thereof, for the detection and/or identification of *Serratia* species.

Another aspect relates to sets of probes for the detection and/or identification of *Serratia* species, in particular of *Serratia marcescens, Serratia ficaria,* and/or *Serratia fonticola* in a sample.

Another aspect concerns primers allowing specific amplification of the 16S-23S rRNA spacer region of *Serratia* species, in particular of *Serratia marcescens, Serratia ficaria,* and/or *Serratia fonticola.*

Another object is a composition containing any of the new sequences of the invention, or any of the new sets of probes and/or primers of the invention, or a combination thereof.

Another object is a kit, in which said probes and/or primers are used, for the detection and/or identification *Serratia* species, in particular of *Serratia marcescens, Serratia ficaria,* and/or *Serratia fonticola.*

Another object is a rapid and reliable hybridization method for detection and/or identification of *Serratia* species, in particular of *Serratia marcescens, Serratia ficaria,* and/or *Serratia fonticola.*

Another object is a hybridization method based on real time PCR for detection and/or identification of *Serratia* species, in particular *Serratia marcescens, Serratia ficaria,* and/or *Serratia fonticola.*

### TABLE LEGENDS

Table 1: Amplification and melting curve program used in the examples.
Table 2: Different combinations of HybProbes tested
Table 3: List of microorganisms tested for specificity of the combination of HybProbes represented by SEQ ID NO 26 and 44.
Table 4: list of SEQ ID NOs 1 to 59.

The SEQ ID's from this table are derived from the following organisms:

| SEQ ID | Organism | SEQ ID | Organism |
|---|---|---|---|
| 1 | *S. marcescens (glu)* | 31 | *S. marcescens (glu)* |
| 2 | *S. marcescens (glu)* | 32 | *S. marcescens (glu)* |
| 3 | *S. marcescens (ile-ala)* | 33 | *SERRATIA (glu + ile-ala)* |
| 4 | *S. marcescens (ile-ala)* | 34 | *S. marcescens (ile-ala)* |
| 5 | *S. marcescens (ile-ala)* | 35 | *S. marcescens (ile-ala)* |
| 6 | *S. ficaria (glu)* | 36 | *S. marcescens (glu + ile-ala)* |
| 7 | *S. ficaria (ile-ala)* | 37 | *S. marcescens (glu + ile-ala)* |
| 8 | *S. fonticola (glu)* | 38 | *SERRATIA (glu + ile-ala)* |
| 9 | *S. fonticola (glu)* | 39 | *SERRATIA (glu + ile-ala)* |
| 10 | *S. fonticola (ile-ala)* | 40 | *S. marcescens (glu + ile-ala)* |
| 11 | *S. fonticola (ile-ala)* | 41 | *S. marcescens (glu + ile-ala)* |
| 12 | *SERRATIA (glu + ile-ala)* | 42 | *S. marcescens (glu + ile-ala)* |
| 13 | *SERRATIA (glu + ile-ala)* | 43 | *S. marcescens (glu + ile-ala)* |
| 14 | *SERRATIA (glu)* | 44 | *S. marcescens (glu + ile-ala)* |
| 15 | *SERRATIA (ile-ala)* | 45 | *S. marcescens (glu + ile-ala)* |
| 16 | *SERRATIA (glu + ile-ala)* | 46 | *S. marcescens (glu + ile-ala)* |
| 17 | *SERRA TIA (ile-ala)* | 47 | *SERRATIA (glu + ile-ala)* |
| 18 | *SERRATIA (ile-ala)* | 48 | *SERRATIA (glu + ile-ala)* |
| 19 | *SERRATIA (ile-ala)* | 49 | *S. marcescens (glu + ile-ala)* |
| 20 | *SERRATIA (ile-ala)* | 50 | *S. marcescens (glu)* |
| 21 | *SERRA TIA (ile-ala)* | 51 | *S. marcescens (glu)* |
| 22 | *SERRA TIA (ile-ala)* | 52 | *S. marcescens (glu)* |
| 23 | *SERRA TIA (ile-ala)* | 53 | *S. marcescens (ile-ala)* |
| 24 | *SERRATIA (ile-ala)* | 54 | *S. marcescens (ile-ala)* |
| 25 | *SERRATIA (ile-ala)* | 55 | *S. marcescens (ile-ala)* |
| 26 | *SERRATIA (glu + ile-ala)* | 56 | *S. marcescens (ile-ala)* |
| 27 | *SERRATIA (ile-ala)* | 57 | *S. marcescens (ile-ala)* |
| 28 | *S. marcescens (ile-ala)* | 58 | *SERRATIA* |
| 29 | *S. marcescens (ile-ala)* | 59 | *SERRATIA* |
| 30 | *S. marcescens (glu)* | | |

### DETAILED DESCRIPTION OF THE INVENTION

The following definitions serve to illustrate the terms and expressions used in the different embodiments of the present invention as set out below.

The terms "spacer" and "ITS" (Internal Transcribed Spacer) are abbreviated terms both referring to the region between the 16S and 23S rRNA or between the 16S and 23S rRNA genes.

The term "probe" refers to a single stranded oligonucleotide or polynucleotide, which has a sequence which is sufficiently complementary to hybridize to a target sequence.

A target sequence in the framework of the present invention is a sequence to be detected comprising any nucleic acid molecule represented by any of the SEQ ID NOs 1 to 11, their complementary form, RNA form thereof, homologues or fragments thereof.

A target sequence can be either genomic DNA or precursor RNA, or amplified versions thereof.

Preferably the probes described are about 80% or more, more preferably about 85% or more, still more preferably about 90% or more and most preferably about 95% or more homologous to the exact complement of the target sequence.

The probes described can be formed by cloning of recombinant plasmids containing inserts including the corresponding nucleotide sequences, if need be by cleaving the latter out from the cloned plasmids using the adequate nucleases and recovering them, e.g. by fractionation according to molecular weight.

The probes described can also be synthesized chemically, for instance by the conventional phospho-triester method.

The term "complementary" nucleic acids as used herein means that the nucleic acid sequences can form a perfect base-paired double helix with each other. If throughout this patent application a complementary form has been mentioned in connection with a sequence and the RNA form thereof then the complementary form of both the sequence and the RNA form thereof is meant.

The terms "polynucleic acid", "nucleic acid", and "polynucleotide" correspond to either double-stranded or single-stranded cDNA or genomic DNA or RNA, containing at least 5, 10, 15, 20, 30, 40 or 50 contiguous nucleotides. A polynucleic acid which is smaller than 100 nucleotides in length is often referred to as an "oligonucleotide". The numbers of nucleotides mentioned in this patent application has to be considered as indicating a range of plus or minus 2 in the same meaning as the term 'about'.

They can also contain modified nucleotides such as inosine or nucleotides containing modified groups which do not essentially alter their hybridization characteristics.

The polynucleic acid molecules described are always represented from the 5' end to the 3' end But they can be used in any form, i.e. their double-stranded or single-stranded form (any of the two strands), their DNA or RNA form (wherein T is replaced by U), modified or not.

The term "closest neighbor" means the taxon which is known or expected to be the most closely related in terms of DNA homology and which has to be differentiated from the organism of interest.

The expression "taxon-specific hybridization" or "taxon-specific probe" means that the probe only hybridizes to the DNA or RNA from the taxon for which it was designed and not to DNA or RNA from other taxa.

The term taxon can refer to a complete genus or a sub-group within a genus, a species or even subtype within a species (subspecies, serovars, sequevars, biovars...).

The term "specific amplification" or "specific primers" refers to the fact that said primers only amplify the relevant region from the organisms for which they were designed, and not from other organisms.

The term "spacer specific amplification" or "spacer specific primers" refers to the fact that said primers only amplify the spacer region from the organisms for which they were designed, and not from other organisms.

The term "specific probe" refers to probes that only hybridize with the relevant region from the organisms for which they were designed, and not with the corresponding region from other organisms, nor with any other region.

The term "spacer specific probe" refers to probes that only hybridize with the relevant spacer from the organisms for which they were designed, and not with spacers from other organisms.

The term "sensitivity" refers to the number of false negatives: i.e. if 1 of the 100 strains to be detected is missed out, the test shows a sensitivity of (100-1/100)% = 99%.

The term "specificity" refers to the number of false positives: i.e. if on 100 strains detected, 2 seem to belong to organisms for which the test is not designed, the specificity of the test is (100-2/100)% = 98%.

The oligonucleotides or polynucleotides selected as being "preferential" show a sensitivity and specificity of more than 80%, preferably more than 90% and most preferably more than 95%.

The term "solid support" can refer to any substrate to which a polynucleotide probe can be coupled, provided that the probe retains its hybridization characteristics and provided that the background level of hybridization remains low. Usually the solid substrate will be a microtiter plate, a membrane (e.g. nylon or nitrocellulose) or a microsphere (bead), without being limited to these examples. Prior to application to the membrane or fixation it may be convenient to modify the nucleic acid probe in order to facilitate fixation or improve the hybridization efficiency. Such modifications may encompass homopolymer tailing, coupling with different reactive groups such as aliphatic groups, NH₂ groups, SH groups, carboxylic groups, or coupling with biotin, haptens or proteins.

The term "labeled" refers to the use of labeled nucleic acids. Labeling may be carried out by the use of labeled nucleotides incorporated during the polymerization step of the amplification such as illustrated by Saiki et al. ((1988) Science 239:487-491) or Bej et al. ((1990) Mol Cell Probes 4:353-365) or by the use of labeled primers, or by any other method known to the person skilled in the art. The nature of the label may be isotopic (³²P, ³¹S, etc.) or non-isotopic (biotin, digoxigenin, fluorescent dye, enzyme, etc.).

The term "signal" refers to a series of electromagnetic waves (for example fluorescence), or changes in electrical current which carry information. The signal can be directly visible, or can be made visible and/or interpretable by different means or devices.

The "sample" may be any biological material. This biological material may be taken either directly from the infected human being, or animal, or after culturing or enrichment, or from food, from the environment, etc.

Biological material may be for example expectoration of any kind, broncheolavages, blood, skin tissue, biopsies, lymphocyte blood culture material, colonies, etc. Said samples may be prepared or extracted according to any of the techniques known in the art.

The *Serratia* species that is clinically relevant in the meaning of the present invention is *Serratia marcescens.*

Different *Serratia* species show two different types of spacer based on the type of tRNA gene inserted in the spacer region, tRNA^{glu} or tRNA^{ile-ala}. Moreover, for each type of spacer and for each *Serratia* species, different clusters or groups can be distinguished.

For instance, out of three strains of S. *marcescens,* having regard to the first type of spacer, i.e. with insertion of tRNA^{glu}, two different groups could be defined, represented respectively by SEQ ID NOs 1 to 2.

Having regard to the second type, i.e. with insertion of tRNA^{ile-ala}, three different groups could be defined, represented respectively by SEQ ID NOs 3 to 5.

To detect and/or identify all *Serratia* species, or each *Serratia* species, or any combination of at least two *Serratia* species, the present disclosure provides new nucleic acid molecules.

An ITS sequence as herein described comprises or consists of a nucleic acid molecule selected from the group consisting of SEQ ID NO 1 to 11, their complementary form, the RNA form thereof wherein T is replaced by U, and any homologous sequences thereof.

Homologous sequences found in the ITS of any *Serratia* species, also referred to herein after as "homologues", are also an object of the present disclosure. The degree of homology relating to the whole sequence is higher than 95%.

In the framework of this disclosure, "homologues" are then homologous sequences to any of SEQ ID NOs 1 to 11 or to any fragment thereof of at least 10, 15, 20, 25, 30, 40, 50, 60, 70, 80, 90, 100 nucleotides, localized in the ITS region of any *Serratia* species and suitable for the detection and/or identification of *Serratia* species.

SEQ ID NOs 1 to 5 are derived from *S*. *marcescens,* SEQ ID NOs 6 and 7 are derived from *S*. *ficaria* and SEQ ID NOs 8 to 11 from *S*. *fonticola.*

New nucleic acid molecules derived from the ITS for the detection of any *Serratia* species, solving the problems generated by a very high variability due to the fact that there are different types of ITS having regard to the tRNA inserted, each type comprising different groups, are provided.

Indeed, it has been discovered that the new nucleic acid molecules consisting of SEQ ID NO 17, 18, 19, 20, 33, 38, 39, and 57 are found in the different groups of a particular type of spacer of every *Serratia* species tested, notably the *Serratia* species that are clinically relevant.

That specific polynucleotides, any fragments thereof of at least 10, 15, 20, 25, 30, and preferably of about 20 nucleotides (18, 19, 20, 21, or 22), the RNA form thereof and the complementary form thereof, also referred as genus-specific polynucleotides, are then specific regions of the ITS that can be used for designing primers and/or probes for the detection of any or all of the *Serratia* species, in particular of the *Serratia* species that are clinically relevant

New polynucleotides for use as probes and/or primers for the detection and/or identification of one, two or more *Serratia* species are provided.

In other words, an object of the disclosure relates to new polynucleotides for use as probes and/or primers, which hybridize with the target sequences of the disclosure for the detection and/or identification of one, two or more *Serratia* species.

In particular, an object of the disclosure is an isolated nucleic acid molecule that specifically hybridizes to a target sequence comprising or consisting of a nucleic acid selected from the group consisting of SEQ ID NO 1 to 11, their RNA form wherein T is replaced by U, the complementary form thereof, any homologues thereof, and fragments of at least about 10, 15, 20, 25, 30, 50, 100, 150, 200, or 300 contiguous nucleotides thereof.

Preferred polynucleotide probes are between about 5 to about 50 bases in length, more preferably from about 10 to about 25 nucleotides and are sufficiently homologous to the target sequence.

Polynucleotides of SEQ IDs NO 12 to 57 or any of their homologues, the complementary form thereof or the RNA form thereof may be used as probes.

Preferred primers are single stranded DNA polynucleotides capable of acting as a point of initiation for synthesis of the target sequence. The length and the sequence of a primer must be such that they allow to prime the synthesis of the extension products.

Preferably a primer is about 5 to about 50 nucleotides long, preferably about 15 to about 25. Its specific length and sequence is to be chosen depending on the conditions used such as temperature and ionic strength.

Primers amplify the target sequences. In other words, primers amplify a nucleic acid molecule comprising any of SEQ ID NOs 1 to 11, their complementary strand and/or homologues.

Universal primers located in the conserved flanking regions of the rRNA spacer, i.e. in the 16S gene and the 23S gene, can be used. If *Serratia* species are present in the sample, the amplification product, the target sequence(s), will then comprise a nucleic acid molecule consisting of any of SEQ ID NOs 1 to 11 and/or homologues.

Preferably, the target sequence(s) consist(s) of any nucleic acid molecules selected from the group consisting of SEQ ID NOs 1 to 11 and/or homologues, flanked by no more than about 40 to about 50 nucleotides of respectively the 16S and 23 S rRNA.

The fact that amplification primers do not have to match exactly with the corresponding template sequence to warrant proper amplification is amply documented in the literature (Kwok et al. (1990) Nucl Acids Res. 18:999).

The amplification method used can be either polymerase chain reaction (PCR; Saiki et al., ((1988), Science 239:487-491), ligase chain reaction (LCR; Landgren et al., ((1988), Science 241:1077-1080), Wu & Wallace, ((1989) Genomics 4:560-569); Barany, ((1991), Proc Natl Acad Sci. USA 88:189-193) nucleic acid sequence-based amplification (NASBA; Guatelli et al., ((1990), Proc Natl Acad Sci. USA 87:1874-1878), Compton, ((1991), Nature 350:91-92) transcription-based amplification system (TAS; Kwoh et al., (1989) Proc Natl Acad Sci. USA 86:1173-1177), strand displacement amplification (SDA; Duck, ((1990) Biotechniques 9:142-147); Walker et al., ((1992) Proc Natl Acad Sci. USA 89:392-396) or amplification by means of QB replicase (Lizardi et al., ((1988) Bio/Technology 6:1197-1202), Lomeli et al., ((1989) Clin Chem 35:1826-1831) or any other suitable method to amplify nucleic acid molecules known in the art.

The preferred polynucleotides for use as primers or as probes, or for designing further primers and probes to be used in methods of the diclosure, are represented by SEQ ID NOs 12 to 57.

Polynucleotides may differ in sequence from any of the polynucleotides represented by SEQ ID NO 12 to 57, either by addition to or removal from any of their respective extremities of one or several nucleotides, or by changing one or more nucleotides within said sequences, or a combination of both, provided that the equivalents then obtained still hybridize with the target sequence. Said equivalent polynucleotides share at least 80% homology, preferably more than 85%, more preferably more than 90% homology, and most preferably more than 95% homology with the corresponding unmodified polynucleotides.

When using an equivalent of a polynucleotide, it may be necessary to modify the hybridization conditions to obtain the same specificity as the corresponding unmodified polynucleotide.

As a consequence, it will also be necessary to modify accordingly the sequence of other polynucleotides when the polynucleotides are to be used in a set under the same hybridization conditions. These modifications can be done according to principles such as those described in Hames B and Higgins S (Eds): Nucleic acid hybridization. Practical approach. IRL Press, Oxford, UK, 1985.

The polynucleotides primers and/or probes of the disclosure may also comprise nucleotide analogues such as phosphorothioates (Matsukura et al., ((1987) Proc Natl Acad Sci. USA 84(21): 7706-7710), alkylphosphorothioates (Miller et al., ((1979), Biochemistry 18(23):5134-5143) or peptide nucleic acids (Nielsen et al., ((1991) Science 254(5037):1497-1500); Nielsen et al., ((1993) Nucl Acids Res. 21(2):197-200) or may contain intercalating agents (Asseline et al., ((1984) Proc Natl Acad Sci. USA 81(11):3297-3301) etc.

The modified primers or probes require adaptations with respect to the conditions under which they are used in order to obtain the required specificity and sensitivity. However the results of hybridization should remain essentially the same as those obtained with the unmodified polynucleotides.

The introduction of these modifications may be advantageous in order to influence some characteristics such as hybridization kinetics, reversibility of the hybrid-formation, biological stability of the polynucleotide molecules, etc.

The probes and primers of the disclosure are used in methods, also objects of the present disclosure, for the detection and/or identification of *Serratia* species, in particular *of Serratia marcescens, Serratia ficaria,* and/or *Serratia fonticola.*

Detection and/or identification of the target sequences can be performed by using an electrophoresis method, a hybridization method or a sequencing method.

A method of the disclosure for the detection of one or more *Serratia* species in a sample comprises the following steps:
- First, and if necessary, the nucleic acids present in the sample are made available for amplification and/or hybridization.
- Secondly, and also if necessary, the nucleic acids, if present, are amplified with one or another target amplification system. Usually, amplification is needed to enhance the subsequent hybridization signal. However for some samples, or for some highly sensitive signal-amplification systems, amplification might not be necessary.
- Thirdly, the nucleic acids present in the sample or the resulting amplified product are contacted with probes, and hybridization is allowed to proceed.
- Finally, the hybrids are detected using a convenient and compatible detection system. From the hybridization signal(s) or pattern(s) observed the presence or absence of one, two or more *Serratia* species can be deduced.

For the amplification step, primers located in the conserved flanking regions (16S and 23S gene) of the rRNA spacer, also called universal primers, can be used. The primers pair represented by SEQ ID NOs 58 and 59 are such examples of universal primers.

For some applications it may be appropriate to amplify not all bacteria present in the sample but one or several genera, or one or several *Serratia* species.

In the latter case, this may be achieved by using genus specific primers or species specific primers derived from the ITS region of *Serratia* species.

In particular, a method of the disclosure for detection and/or identification of *Serratia* species in a sample comprises the steps of:
(i) optionally, isolating and/or concentrating the polynucleic acids present in the sample;
(ii) optionally amplifying the 16S-23S rRNA spacer region(s), or at least one of the target sequences or (a) fragment(s) thereof, with at least one suitable primer pair;
(iii) contacting the polynucleic acids with at least one polynucleotide probe that hybridizes to at least one of the target sequences selected from the group consisting of SEQ ID NOs 1 to 11, homologues thereof, their RNA form wherein T is replaced by U, the complementary form thereof and fragments thereof;
(iv) detecting the hybrids formed, and
(v) interpreting the signal(s) obtained and inferring the presence of *Serratia* species and/or identifying the *Serratia* species in the sample.

A fragment, as mentioned for instance in the amplification or the hybridization step of any method of the disclosure, may comprise or consist of about 10, 15, 20, 25, 30, 50, 100, 200, 300 contiguous nucleotides of a nucleic acid molecule of the disclosure.

Preferably, the probes of the disclosure hybridize under conditions of high stringency.

Under high stringency conditions only complementary nucleic acid hybrids are formed. Accordingly, the stringency of the assay conditions determines the amount of complementarity needed between two nucleic acid strands forming a hybrid. Stringency is chosen to maximize the difference in stability between the hybrid formed with the target and the non-target nucleic acid.

In any case, the appropriate hybridization conditions are chosen in such a way that the signal of hybridization obtained when a polynucleotide of the disclosure hybridizes specifically to a target sequence, is different from the signal obtained when said polynucleotide hybridizes to a target sequence in a non-specific manner.

In practice, the different signals may be visualized for example when its intensity is two, five, ten or more times stronger with a specific hybridization to the target, as compared to non-specific hybridization to the target sequence. The Line Probe Assay (LiPA) system is a good example in this respect.

The different signals may also be visualized when different peaks are drawn in a melting curve analysis, for instance when using a real time PCR method.

In one embodiment, a very convenient and advantageous technique for the detection of target sequences that are possibly present in the sample is the real time PCR method.

There are different formats for the detection of amplified DNA that can be used in the framework of the present invention, notably TaqMan™ probes, Molecular Beacons probes, "Scorpions", or FRET hybridization probes.

Concerning the TaqMan™ probes, a single-stranded hybridization probe is labeled with two components. When the first component, the so-called fluorescer, is excited with light of a suitable wavelength, the absorbed energy is transferred to the second component, the so-called quencher, according to the principle of fluorescence resonance energy transfer. During the annealing step of the PCR reaction, the hybridization probe binds to the target DNA and is degraded by the 5'-3' exonuclease activity of the polymerase, for example Taq Polymerase, during the elongation phase. As a result the excited fluorescent component and the quencher are spatially separated from one another and thus a fluorescence emission of the first component can be measured (EPB 0543 942 and US 5,210,015).

Concerning Molecular Beacons probes, the probes are also labeled with a first component and with a quencher, the labels preferably being located at different ends of an at least partially self-complementary probe. As a result of the secondary structure of the probe, both components are in spatial vicinity in solution. After hybridization to the target nucleic acids both components are separated from one another such that after excitation with light of a suitable wavelength the fluorescence emission of the first component can be measured (US 5,118,801).

Concerning "Scorpions", a probe and a primer are contained in one molecule. Similarly to Molecular Beacons system, each probe is labeled with a first component and with a quencher, the labels being located at different ends of an at least partially self-complementary probe. A primer is linked to each probe by the intermediary of a PCR stopper which prevents the secondary structure from being opened in the absence of the specific target sequence. (Whitcombe, D. et al., (1999) Nature Biotechnology 17: 804-807 ; Thelwell, N. et al., (2000) Nucleic Acids Research vol. 28, No 19: 3752-3761; Svanvik et al Analytical Biochemistry 287: 179-182 (2000)).

The Fluorescence Resonance Energy Transfer (FRET) hybridization probe test format is especially useful for all kinds of homogenous hybridization assays (Matthews, J. A. and Kricka, L. J., (1988) Anal Biochem 169: 1-25). It is characterized by two single-stranded hybridization probes which are used simultaneously and are complementary to adjacent sites of the same strand of an (amplified) target nucleic acid. Both probes are labeled with different fluorescent components. When excited with light of a suitable wavelength, a first component transfers the absorbed energy to the second component according to the principle of fluorescence resonance energy transfer such that a fluorescence emission of the second component can be measured only when both hybridization probes bind to adjacent positions of the target molecule to be detected.

When annealed to the target sequence, the hybridization probes must be located very close to each other, in a head to tail arrangement. Usually, the gap between the labeled 3' end of the first probe and the labeled 5' end of the second probe is as small as possible, and notably consists of about 0 to 25 bases, and preferably of about 1 to about 5 bases. This allows for a close vicinity of the FRET donor compound and the FRET acceptor compound, which is typically 10-100 Angstrom.

Alternatively to monitoring the increase in fluorescence of the FRET acceptor component, it is also possible to monitor fluorescence decrease of the FRET donor component as a quantitative measurement of hybridization event.

Among all detection formats known in the art of real time PCR, the FRET-hybridization probe format has been proven to be highly sensitive, exact and reliable (WO 97/46707; WO 97/46712; WO 97/46714). Yet, the design of appropriate FRET hybridization probe sequences may sometimes be limited by the special characteristics of the target nucleic acid sequence to be detected.

As an alternative to the usage of two FRET hybridization probes, it is also possible to use a fluorescent-labeled primer and only one labeled polynucleotide probe (Bernard, P. S., et al., (1998) Anal. Biochem. 255: 101-7). In this regard, it may be chosen arbitrarily, whether the primer is labeled with the FRET donor or the FRET acceptor compound.

The fluorescence can be measured during the elongation step, generating amplification curves from which, depending on the primers and/or probes used, on their Tm and on the hybridization conditions, it is possible to infer the presence of the *Serratia* species to be detected or to infer which *Serratia* species is (are) present.

FRET hybridization probes (also called HybProbes or FRET-probes) can also be used for melting curve analysis (WO 97/46707; WO 97/46712; WO 97/46714). In such an assay, the target nucleic acid is amplified first in a typical PCR reaction with suitable amplification primers. The hybridization probes may already be present during the amplification reaction or be added subsequently. After completion of the PCR-reaction, the temperature of the sample is consecutively increased. Fluorescence is detected as long as the hybridization probe is bound to the target DNA. At the melting temperature, the hybridization probe is released from their target, and the fluorescent signal is decreasing immediately down to the background level. This decrease is monitored with an appropriate fluorescence versus temperature-time plot such that the negative of a first derivative function can be calculated. The temperature value corresponding to the obtained maximum of such a function is then taken as the determined melting temperature of said pair of FRET hybridization probes.

Point mutations or polymorphisms within the target nucleic acid result in a less then 100% complementarity between the target nucleic acid and the FRET probes, thus resulting in a decreased melting temperature. This enables for a common detection of a pool of sequence variants by means of FRET-HybProbe hybridization, whereas subsequently different members of said pool may become discriminated by means of performing melting curve analysis.

Instead of FRET hybridization probes, Molecular Beacons may alternatively be used for melting curve analysis.

Upon the availability of Real-Time PCR and homogenous Real-Time PCR melting curve analysis, discrimination of certain types of species or strains became possible using either double stranded DNA binding dyes such as SybrGreen™I, or, alternatively, specifically designed hybridization probes hybridizing to different but similar target sequences.

In the first case, melting temperature of the generated double stranded PCR product has to be determined. Yet, this method has only limited applications since few differences cannot be monitored efficiently, because minor sequence variations only result in subtle melting temperature differences.

Alternatively, hybridization probes may be used in such a way that the melting temperature of the probe/target nucleic acid hybrid is being determined.

There are different real time PCR platforms that can be used, such as the ABI/Prism™ equipments, and in particular the LightCycler™ apparatus, all based on the same principle consisting of measuring the light emission, continually monitoring the emission peak during the melt cycle, determining and visualizing the temperatures (melting peaks) at which the labeled probes detach from the amplification products. The melting peak data are characteristic of a particular [probe:target] sequence because mismatches between probe and target affect the kinetics of melting, producing different melting peaks for each species of interest.

The LightCycler™ platform offers many advantages and in particular a gain of time and the possible use of several different sequence-specific fluorescent probe detection systems such as hybridization probes (HybProbes), TaqMan™ probes, Molecular Beacons, Scorpion probes and biprobes (SybrGreen™I).

In a preferred method of the present disclosure, the HybProbe system is used, consisting of two adjacent polynucleotide probes derived from the target sequences of the invention, in a head-to-tail orientation, spaced by a few nucleotides, generally 0 to 25, preferably about 1 to about 5. One of the probes is labeled at its 3' end by a donor dye, the other is labeled with an acceptor molecule at its 5' end, and is phosphate blocked at the 3' end (to prevent its acting as a primer). The donor dye is generally fluorescein, and the acceptor molecule generally LC Red 610, 640, 670 or 705.

The detection of a target sequence of the disclosure may be achieved also by an internal labeled PCR strand and a detection probe located on the opposite strand. The signal is dependent on the spatial approximation of the dyes, and is dependent on the amount of the target.

When both probes are hybridized to their target sequence the emitted light of the donor is transmitted to the acceptor fluorophore by Fluorescence Resonance Energy Transfer (FRET), and the emitted fluorescence (610, 640, 670 or 705 nm) can be detected The intensity of the emitted fluorescence increases in parallel with the target DNA, product of the amplification.

The LightCycler™ probes offer the advantage over the TaqMai™ probes of not requiring hydrolysis and, therefore, no additional extension of the PCR times (annealing-elongation ≤ 12 s). It is therefore possible to take advantage of the high-speed thermal cycling of the LightCyler™, and complete the PCR program in only 45 minutes.

And the recent generations of real-time PCR platforms are able to monitor several probes in a single reaction, allowing the detection and/or identification of different *Serratia,* at the species level and/or the distinction of the different type of *Serratia* spacers.

Moreover, it has been shown that the methods designed for TaqMan™ technology can be easily converted to HybProbe technology with equivalent results (Haematologica vol. 85 (12) pp. 1248-1254, December 2000).

Therefore another object relates to sets of at least two polynucleotide probes, also referred to as HybProbes, both HybProbes hybridizing to the same target sequence, adjacent to each other, with no more than 25 nucleotides between said two HybProbes, preferably with no more than 10 nucleotides, in particular with no more than 5 nucleotides.

When there are two HybProbes, one is labeled with an acceptor fluorophore and the other with a donor such that upon hybridization of the two HybProbes with the target sequence, the donor and acceptor fluorophores are within 0 to 25 nucleotides of one another, and preferably within 0 to 5 nucleotides of one another.

When there are more than two HybProbes, at least one is labeled with an acceptor fluorophore and the others with a donor (or vice versa) such that upon hybridization of the HybProbes with the target sequence, the donor and acceptor fluorophores are within 0 to 25 nucleotides of one another, and preferably within 0 to 5 nucleotides of one another.

For detecting and/or identifying *Serratia* species, in particular *Serratia* species that are clinically relevant, a set of at least two polynucleotide probes may be used, said probes hybridizing with at least one of the target sequences selected from the group consisting of SEQ ID NOs 1 to 11, their RNA form wherein T is replaced by U, the complementary form thereof, and homologues, wherein there are no more than 25 nucleotides, preferably no more than 5 nucleotides, between said probes.

A set of probes may also consist of 3, 4, 5, 6, 7, 8, 9, 10, or more, probes, but it preferably consists of 2 to 5 probes.

The sets of probes listed in Table 2 and their homologues are preferred sets of the disclosure.

Sets of three polynucleotides, two for use as primer, the other for use as probe, may also be used. Then one of said primers and the said probe hybridize to at least one of the target sequences selected from the group consisting of SEQ ID NOs 1 to 11, their RNA form wherein T is replaced by U, the complementary form thereof, and homologues, so that there are no more than 25 nucleotides, preferably no more than 5 nucleotides, between said primer and said probe.

The sets of at least two polynucleotides of the disclosure are used in methods for the detection and/or identification of *Serratia* species, in particular of *S*. *marcescens, S. ficaria* and/or *S*. *fonticola.*

A method of the present disclosure for detection and/or identification of *Serratia* species in a sample, in particular of *S*. *marcescens, S. ficaria* and/or *S*. *fonticola,* comprises the steps of:
(i) optionally, releasing, isolating and/or concentrating the polynucleic acids in the sample;
(ii) amplifying the 16S-23S rRNA spacer region, or at least one target sequence, or a fragment thereof, with at least one suitable primer pair;
(iii) contacting the polynucleic acids with at least one set of at least two HybProbes that hybridize to at least one target sequence selected from the group consisting of SEQ ID NOs 1 to 11, their RNA form wherein T is replaced by U, the complementary form thereof, any homologues, or a fragment of at least 10, preferably of at least 15, more preferably of at least 20 contiguous nucleotides thereof;
(iv) detecting the hybrids formed in step (iii);
(v) inferring the presence of *Serratia* species, or identifying the *Serratia* species in the sample from the differential hybridization signals obtained in step (iv).

For example, a set of two HybProbes used in the hybridization step can be any combination of the HybProbe represented by SEQ ID NO 26 with any of the HybProbes represented by SEQ ID NOs 44 and 45, or their homologues.

The HybProbe represented by SEQ ID NO 26 can be fluorescein labeled and the others can be either LCR610, LCR640, LCR670 or LCR705 labeled.

One of the advantages of the HybProbes system resides in the fact that it allows the detection of sequence variation, including mutations, polymorphisms and other variant nucleic acid species, based on the following molecular concept: one of the HybProbe is a tightly binding "anchor probe" whereas the adjacent "sensor probe" spans the region of sequence variation. During melting of the final PCR product, the sequence alteration is detected as a change in the melting temperature (Tm) of the sensor probe.

For example, if the sample contains only SEQ ID NO 1, using HybProbes that specifically hybridize to said SEQ ID NO 1 would generate a single melting peak. If there is also a homologue in the sample, using the same two HybProbes would generate two peaks, as far as there is at least one mismatched base which generally induces a temperature shift easily observable.

Depending on the format of the probes used for the detection of the products of the amplification, on the polynucleotides selected (or designed), on their Tm and on the hybridization conditions, the fluorescence may be measured during the amplification step, generating then amplification curves, or after the amplification step, for a melting curve analysis, generating melting curves.

Thus the signal(s) obtained may be visualized in the form of amplification curves or in the form of melting curves, from which it is possible to infer the presence of *Serratia* species, and/or to infer which one(s) of the *Serratia* species is/are present.

In particular, a method for detection and/or identification of *Serratia* species in a sample comprises also the steps of
(i) if need be releasing, isolating and/or concentrating the polynucleic acids in the sample, and
(ii) amplifying at least one of the target sequences selected from the group consisting of SEQ ID NO 1 to 11, their RNA form wherein T is replaced by U, the complementary form thereof, any homologues, or a fragment of at least 10, preferably of at least 15, more preferably of at least 20 contiguous nucleotides thereof, with a pair of primers one of which is labeled,
(iii) contacting the polynucleic acids with at least one HybProbe that hybridize, adjacent to said labeled primer with less than 25 nucleotides in between, to said target sequence(s),
(iv) detecting the hybrids formed, and
(v) inferring the presence of *Serratia* species, and/or identifying the *Serratia* species in the sample from the signals obtained in step (iv).

A method of the disclosure using the HybProbes system, may be adapted for the detection and identification of one or several *Serratia* species, allowing its/their distinction from other *Serratia* species.

In particular, a method of the disclosure using the HybProbes system, may be adapted for the detection and identification of *Serratia marcescens,* allowing its distinction from other *Serratia* species.

Then, in the amplification step, suitable primers are primer pairs that specifically amplify the target sequence(s) selected from a group consisting of SEQ ID NOs 1 to 5, their RNA form wherein T is replaced by U, the complementary form thereof and homologues.

In the hybridization step, the HybProbes should hybridize specifically for example to any of SEQ ID NO 12 to 14, 21 to 23, 26, 27 to 32, 40 to 46, 49 and 50 to 56 or to their RNA form wherein T is replaced by U, or to the complementary form thereof.

Therefore, *Serratia marcescens* and *Serratia ficaria* strains can be unequivocally distinguished from all other organisms examined by melting curve analysis.

No relevant signals are obtained with *non-Serratia* species or human genomic DNA.

A preferred set of two HybProbes consists of SEQ ID NO 26 or homologues and SEQ ID NO 44 or 45 or homologues.

This set of HybProbes consisting of SEQ ID NO 26 and 44 or 45 is able to detect *Serratia marcescens* with a high sensitivity.

Each polynucleotide listed in Table 4, corresponding to SEQ ID NO 12 to SEQ ID NO 57 and any of their homologues, may be used in any methods of the present disclosure as a primer and/or as a probe, alone or in combination.

A second embodiment based also on a hybridization method is the Line Probe Assay technique. The Line Probe Assay (LiPA) is a reverse hybridization format (Saiki et al., (1989), Proc Natl Acad Sci U S A. 16:6230-4) using membrane strips onto which several polynucleotide probes (including negative or positive control polynucleotides) can be conveniently applied as parallel lines.

The LiPA technique, as described by Stuyver et al. ((1993), J Gen Virol. 74 (Pt 6):1093-102) and in EPB 0637342, provides a rapid and user-friendly hybridization test. Results can be read within 4 h. after the start of the amplification. After amplification during which usually a non-isotopic label is incorporated in the amplified product, and alkaline denaturation, the amplified product is contacted with the probes on the membrane and the hybridization is carried out for about 1 to 1,5 h. Consequently, the hybrids formed are detected by an enzymatic procedure resulting in a visual purple-brown precipitate. The LiPA format is completely compatible with commercially available scanning devices, thus rendering automatic interpretation of the results possible. All those advantages make the LiPA format suitable for application in a routine setting.

The LiPA format is an advantageous tool for detection and/or identification of pathogens at the species level but also at higher or lower taxonomical levels. For instance, probe-configurations on LiPA strips can be selected in such a manner that they can detect the complete genus of *Serratia* or can identify species within the genus (e.g. *S*. *marcescens, S. ficaria* and/or S. *fonticola,* etc) or can in some cases even detect subtypes within a species.

The ability to simultaneously generate hybridization results with a large number of probes is another benefit of the LiPA technology. In many cases the amount of information which can be obtained by a particular combination of probes greatly outnumbers the data obtained by using single probe assays. Therefore the selection of probes on the membrane strip is of utmost importance since an optimized set of probes will generate the maximum of information possible.

These probes can be applied to membrane strips at different locations and the result is interpreted as positive if at least one of these probes is positive. Alternatively these probes can be applied as a mixture at the same location, hereby reducing the number of lines on a strip. This reduction may be convenient in order to make the strip more concise or to be able to extend the total number of probes on one strip.

Another approach is the use of degenerate probes, which can considerably simplify the manufacturing procedures of the LiPA-strips.

By virtue of the above-mentioned properties the LiPA system can be considered as an efficient format for a hybridization method wherein several organisms need to be detected simultaneously in a sample.

However, it should be clear that any other hybridization assay, whereby different probes are used under the same hybridization and wash conditions can be used for the above-mentioned detection and/or selection methods. For example, it may be possible to immobilize the target nucleic acid to a solid support, and use mixtures of different probes, all differently labeled, resulting in a different detection signal for each of the probes hybridized to the target. And nowadays many different supports are available.

As an example, the procedure to be followed for the detection of one or more *Serratia* species in a sample using the LiPA format is outlined below:
- First, and if necessary, the nucleic acids present in the sample are made available for amplification and/or hybridization.
- Optionally, the nucleic acids are amplified with one or another target amplification system. Usually, amplification is needed to enhance the subsequent hybridization signal.
- Thirdly, eventually after a denaturation step, the nucleic acids present in the sample or the resulting amplified product are contacted with LiPA strips onto which one or more probes, allowing the detection of the organisms of interest, are immobilized, and hybridization is allowed to proceed.
- Finally, eventually after having performed a wash step, the hybrids are detected using a convenient and compatible detection system. From the hybridization signal(s) or pattern(s) observed the presence or absence of one or several organisms screened for in that particular biological sample can be deduced.

Universal primers located in the conserved flanking regions of the rRNA spacer, i.e. in the 16S gene and the 23S gene, can be used.

For some applications it may be appropriate to amplify not different bacteria present in the sample but more specifically *Serratia* species.

A method of the disclosure for detection and/or identification of *Serratia* species in a sample, comprises the steps of:
(i) if need be releasing, isolating and/or concentrating the polynucleic acids present in the sample;
(ii) if need be amplifying the 16S-23S rRNA spacer region, or a part of it, with at least one suitable primer pair;
(iii) contacting the polynucleic acids with at least one probe that hybridizes to the target sequence consisting of SEQ ID NO 1 to 11, or of the RNA form of said SEQ ID NO 1 to 11 wherein T is replaced by U, or of the complementary form thereof, or of any homologues, or of a fragment of at least 10, preferably of at least 15, more preferably of at least 20 contiguous nucleotides thereof;
(iv) detecting the hybrids formed in step (iii);
(v) detecting and/or identifying the micro-organism(s) present in the sample from the differential hybridization signals obtained in step (iv).

The part of the ITS mentioned in the step of amplification, is a polynucleotide comprising the target sequence, or the target sequence itself, the target sequence consisting of any of SEQ ID NO 1 to 11, or of their RNA form wherein T is replaced by U, or of the complementary form thereof, or of any homologues, or of a fragment of at least 10, preferably of at least 15, more preferably of at least 20 contiguous nucleotides thereof.

Preferentially, the present disclosure provides for a method as described above wherein at least two micro-organisms are detected simultaneously. In a preferred method of the invention, in step (iii) of the process as described above a set of at least two probes is used. Such a set of probes as described in step (iii) comprises at least two, three, four, five, six, seven, eight, nine or more probes of the invention.

Preferred probes are polynucleotides of SEQ ID NO 12 to 57, their RNA form wherein T is replaced by U, the complementary form thereof, any homologues, and fragments of at least 10, preferably of at least 15 and more preferably of at least 20 contiguous nucleotides thereof.

The present disclosure also provides for a method as described above, wherein the probes as specified in step (iii) are combined with at least one probe of another microorganism, preferentially also from the 16S-23S rRNA spacer region, enabling the simultaneous detection of different pathogenic bacteria liable to be present in the same sample.

Preferred probes are designed for attaining optimal performance under the same hybridization conditions so that they can be used in sets for simultaneous hybridization; this highly increases the usability of these probes and results in a significant gain in time and labor.

A kit containing any of the polynucleotides of the present disclosure is also an object of the disclosure.

A kit of the disclosure comprises the following components:
- at least one polynucleotide hybridizing to the target sequence consisting of any of SEQ ID NO 1 to 11, their RNA form wherein T is replaced by U, the complementary form thereof, or homologues thereof, or a fragment of at least 10, preferably of at least 15, more preferably of at least 20 contiguous nucleotides thereof, for the detection and identification of *Serratia* species;
- a hybridization buffer, or components necessary for producing said buffer.

A preferred kit comprises
- at least one set of two HybProbes, adjacent to each other with less than 25 nucleotides, preferably less than 5 nucleotides, hybridizing to the target sequence consisting of any of SEQ ID NO 1 to 11, their RNA form wherein T is replaced by U, the complementary form thereof, or any homologues thereof or a fragment of at least 10, preferably of at least 15, more preferably of at least 20 contiguous nucleotides thereof, for the detection and identification of *Serratia* species;
- a hybridization buffer, or components necessary for producing said buffer.

To conclude, using the *Serratia* ITS as target, it is possible to design probes to be used in different detection and/or identification methods.

With the real time PCR method, on the one hand it is possible to detect and identify the *Serratia* genus - in particular *S*. *marcescens, S. ficaria,* and *S. fonticola -* using one single HybProbe set generating one single melting peak in the LightCycler^{™} system (example 4).

On the other hand, a species-specific signal can be obtained by the presence of one specific melting peak for one or more particular species *(S. marcescens* and *S. ficaria* in example 3), or by the presence of a peak at a Tm that is specific for a particular species (see other *Serratia spp.* in example 3).

Also sequencing the complete ITS region and comparing it to a reference sequence as given here, can be used as a method to detect and identify *Serratia* species (example 5).

The preceding description or the examples which follow should not be construed as limiting the invention to the embodiments specifically disclosed therein.

### EXAMPLES

For the examples described below, the 16S-23S internal transcribed spacer (ITS) was amplified using primers designed in conserved regions of the 16S rRNA and 23S rRNA, respectively.

### Example 1: LightCycler^{™} protocol

DNA was prepared according to standard methods, and about 10⁴ genome equivalents were used as target for amplification.

A sample was flagged positive if a quantification curve and a melting peak were present for that sample.

The probes were designed to work as HybProbes in the LightCycler^{™} v1.2 (software v4) enabling a real-time fluorescence PCR detection.

One HybProbe was labeled at its 3' end with a fluorescein dye, while the neighboring HybProbe was labeled at its 5' end with a LC-red 640 or LC-red 705 dye.

Following the instructions of the manufacturer of the kit LC-FastStart DNA Master Hybridization Probes (cat. No 3 003 248 or No 2 239 272):
- any sample material suitable for PCR in terms of purity, concentration, and absence of inhibitors can be used ;
- the primers should be at a final concentration of 0,3 to 1 µM each ;
- the HybProbes at a final concentration of 0,2 µM each, or double;
- the concentration of MgCl₂ should be optimized, and may vary from 1 to 5mM;
- and a negative control should be run.

The amplification and melting conditions are described herein after. The LC software version 4 was used. The quantification settings were F2/back F1 (samples). For the baseline adjustment the arithmetic mode was used. The crossing point (Ct) calculation was based on the second derivative maximum. The calculation method for the melting peak was polynomial. The peak area was used to calculate the Tm.

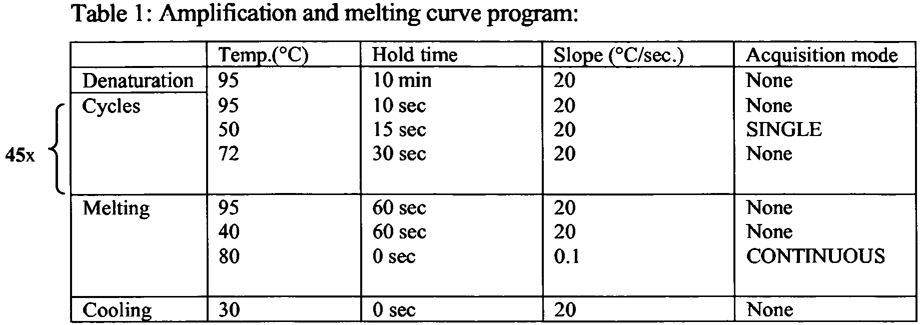

### Example 2: Different sets of Hybprobes

In this example, one HybProbe was labeled at its 3' end with a fluorescein dye, while the neighboring HybProbe was labeled at its 5' end with LC-Red 640 or LC-Red 705 dye.

The same LightCycler^{™} protocol as described in example 1 was applied.

Different probe combinations were evaluated using genomic DNA of *Serratia* species and other clinically relevant bacteria. First selection criteria for preferred probe combinations were: quality of fluorescent signal resulting in quantification curves and nic melting peaks; detection of *Serratia marcescens* and/or *Serratia* species at the same Tm and lack of cross reaction with other bacteria.

**Table 2: Results of different combinations tested**

| SEQ ID NOs Fluorescein labeled | SEQ ID Nos LC-Red labeled | Design goal | Strains detected / strains tested | | | | Preferred / most preferred |
|---|---|---|---|---|---|---|---|
| | | | *S. marcescens* | *S. ficaria* | *Serratia spp.* | Other bacteria | |
| 12 | 27 | *S. marcescens* specific | 1/1 | - | - | - | + |
| 13 | 28 | *S. marcescens* specific | 9/9 | 1/1 | 2/4 | - | + |
| 13 | 29 | *S. marcescens* specific | 16/16 | 1/1 | - | - | + |
| 14 | 30 | *S. marcescens* specific | 11/20 | 0/1 | 1/4 | - | (+) |
| 14 | 31 | *S. marcescens* specific | 12/21 | 0/1 | 1/4 | - | (+) |
| 14 | 32 | *S. marcescens* specific | 21/21 | 0/1 | 1/4 | - | (+) |
| 15 | 33 | *Serratia* genus | 9/9 | - | - | - | + |
| 16 | 34 | *Serratia* genus | 9/9 | - | - | - | + |
| 16 | 35 | *Serratia* genus | 9/9 | - | - | - | + |
| 17 | 36 | *Serratia* genus | 4/4 | 1/1 | 1/1 | - | + |
| 17 | 37 | *Serratia* genus | 4/4 | 1/1 | 1/1 | - | + |
| 17 | 38 | *Serratia* genus | 4/4 | 1/1 | 1/1 | - | + |
| 18 | 36 | *Serratia* genus | 4/4 | 1/1 | 1/1 | - | + |
| 18 | 37 | *Serratia* genus | 4/4 | 1/1 | 1/1 | - | + |
| 18 | 38 | *Serratia* **genus** | 4/4 | 1/1 | 1/1 | - | + |
| 19 | 36 | *Serratia* genus | 4/4 | 1/1 | 1/1 | - | + |
| 19 | 37 | *Serratia* genus | 42/42 | 1/1 | 1/1 | 0/19 | ++ |
| 19 | 38 | *Serratia* genus | 4/4 | 1/1 | 1/1 | - | + |
| 17 | 33 | *Serratia* genus | 4/4 | 1/1 | 1/1 | - | + |
| 18 | 33 | *Serratia* genus | 4/4 | 1/1 | 1/1 | - | + |
| 19 | 33 | *Serratia* genus | 4/4 | 1/1 | 1/1 | - | + |
| 19 | 39 | *Serratia* genus | 1/1 | 1/1 | 2/2 | 0/1 | + |
| 20 | 37 | *Serratia* genus | 42/42 | 1/1 | 4/4 | 0/1 | ++ |
| 20 | 39 | *Serratia* genus | 7/7 | 1/1 | 2/2 | 0/1 | + |
| 24 | 47 | *Serratia* genus | 1/1 | 1/1 | 2/2 | 0/1 | + |
| 25 | 47 | *Serratia* genus | 7/7 | 1/1 | 2/2 | 0/1 | + |
| 25 | 48 | *Serratia* genus | 42/42 | 1/1 | 4/4 | 0/1 | ++ |
| 21 | 40 | *S. marcescens* specific | 7/7 | 1/1 | 2/2 | - | (+) |
| 21 | 41 | *S. marcescens* specific | 7/7 | 1/1 | 1/2* | - | ++ |
| 21 | 44 | *S. marcescens* specific | 1/1 | 1/1 | 2/2* | - | + |
| 21 | 49 | *S. marcescens specific* | 11/11 | 1/1 | 2/2* | - | ++ |
| 22 | 40 | *S. marcescens* specific | 11/11 | 1/1 | 2/2 | - | (+) |
| 22 | 41 | *S. marcescens* specific | 11/11 | 1/1 | 2/2* | - | ++ |
| 22 | 44 | *S. marcescens* specific | 24/24 | 1/1 | 4/4* | 0/28 | ++ |
| 22 | 46 | *S. marcescens* specific | 1/1 | 1/1 | 2/2* | - | + |
| 22 | 49 | *S. marcescens* specific | 7/7 | 1/1 | 1/2* | - | ++ |
| 23 | 42 | *S. marcescens* specific | 6/6 | 1/1 | 2/2* | - | ++ |
| 23 | 43 | S. *marcescens* specific | 6/6 | 1/1 | 2/2* | - | ++ |
| 23 | 44 | *S. marcescens* specific | 1/1 | 1/1 | 2/2* | - | + |
| 23 | 46 | *S. marcescens* specific | 1/1 | 1/1 | 2/2* | - | + |
| 13 | 46 | *S. marcescens* specific | 1/1 | 1/1 | 2/2* | - | + |
| 26 | 44 | *S. marcescens* specific | 42/42 | 1/1 | 4/4* | 0/56 | ++ |
| 26 | 45 | *S*. *marcescens* specific | 42/42 | 1/1 | 4/4* | 0/56 | ++ |
| 26 | 46 | *S. marcescens* specifics | 1/1 | 1/1 | 2/2* | 0/1 | + |
| 26 | 49 | *S. marcescens* specific | 1/1 | 1/1 | 2/2* | 0/1 | + |
| | | | | | * = detected by peak with lower Tm | | |

### Example 3: HybProbes for distinguishing S. marcescens and S. ficaria from other Serratia species.

The HybProbes represented by SEQ ID NO 26 and SEQ ID NO 44 were used in a LightCycler™ protocol as described in example 1. The first (SEQ ID NO 26) was fluorescein labeled and the second (SEQ ID NO 44) was LC-Red 640 labeled.

The same LightCycler™ protocol as described in example 1 was applied, and the sample used contained one of the *S. marcescens* strains. One specific melting peak at 58°C was observed.

The sensitivity of this HybProbe set was evaluated using 42 *S. marcescens* strains (10 originating from West-Europe, 10 from the UK, 10 from South-Europe, 10 from the United States, and 2 from Japan). All *S. marcescens* strains had a visible quantification curve with Ct values varying from 20.6 to 31.0.

A melting peak of 58°C (STDEVA 0.29°C) was observed for *S. marcescens* strains tested, showing a 100% sensitivity for *S. marcescens* with this HybProbe set.

In order to test specificity other *Serratia* species and close neighbours were tested. *S. marcescens* strains cannot be distinguished from *S. ficaria* with this HybProbe set resulting in a same melting peak at 58°C for the latter species. It should be noted that the clinical relevance of *S. ficaria* is very low, and that this species is rarely found in clinical samples. The other *Serratia* species had a melting peak at 55°C to 56°C, and could hereby be differentiated from *S. marcescens.* Closely related species like *Yersinia pseudotuberculosis* and *Y. enterocolitica* had lower melting peaks at 46°C to 47°C.

Besides these *Serratia* species and close relatives, a large panel of other organisms was tested (see Table 3) and a further experiment was done with human DNA. Neither the human DNA nor the microorganisms tested gave any quantification curve or any melting peak.

**Table 3: list of microorganisms tested for specificity.**

| | |
|---|---|
| *Acinetobacter baumannii* | *Bartonella henselae* |
| *Aspergillus fumigatus* | *Bordetella pertussis* |
| *Candida albicans* | *Borrelia burgdorferi* |
| *Candida glabrata* | *Burkholderia cepacia* |
| *Candida krusei* | *Campylobacter jejuni* |
| *Candidia paravsilosis* | *Cardiobacterium hominis* |
| *Candida tropicalis* | *Citrobacter freundii* |
| *Enterobacter aerogenes* | *Clostridium perfringens* |
| *Enterobacter cloacae* | *Corynebacterium jeikeium* |
| *Enterococcus faecalis* | *Cryptococcus neoformans* |
| *Enterococcus faecium* | *Gemella haemolysans* |
| *Escherichia coli* | *Histoplasma capsulatum* |
| *Klebsiella oxytoca* | *Haemophilus influenzae* |
| *Klebsiella pneumoniae* | *Legionella pneumophila* |
| *Pseudomonas aeruginosa* | *Listeria monocytogenes* |
| *Proteus mirabilis* | *Moraxella (Branhamella) catarrhalis* |
| *Staphylococcus aureus* | *Morganella morganii* |
| *Staphylococcus epidermidis* | *Mycobacterium fortuitum* |
| *Staphylococcus haemolyticus* | *Mycobacterium tuberculosis* |
| *Stenotrophomonas maltophilia* | *Mycoplasma pneumoniae* |
| *Streptococcus sanguinis "Sanguinis"* group | *Neisseria meningitidis* |
| *Streptococcus agalactiae* | *Pantoea agglomerans* |
| *Streptococcus pneumoniae* | *Peptostreptococcus magnus* |
| *Streptococcus pyogenes* | *Porphyromonas gingivalis* |
| *Actinobacillus actinomycetemcomitans* | *Prevotella denticola* |
| *Aeromonas hydrophila* | *Propionibacterium acnes* |
| *Bacillus cereus* | *Salmonella enterica v.enteritidis* |
| *Bacterioides fragilis* | |

### Example 4: HybProbes for Serratia species

Five samples containing respectively one strain of *S. marcescens,* one of *S. ficaria,* one of *S. fonticola,* one of *S*. *grimessi* and one of *S. proteamaculans* were tested.

The HybProbes represented by SEQ ID NO 20 and SEQ ID NO 37 were used in a LightCycler™ protocol as described in example 1.

Each strain generated a quantification curve and one melting peak at 56°C was observed.

Closely related genera, like *Yersinia* species did not generate any quantification curve nor melting peak, indicating that the HybProbe set used is specific for *Serratia* spp.

### Example 5: Detection and identification of Serratia spp. by ITS nucleotide sequence determination.

A sample was received without a clear indication of the *Serratia* species it was supposed to contain.

The ITS region of the species to be determined was amplified using universal primers located in the 16S and 23S region.

The amplicons were cloned into the pGEM-T vector (Promega) and the ITS nucleotide sequences were derived according to the dideoxy-chain terminating chemistry using primers located in the plasmid vector.

Both a spacer containing tRNA^{glu} and tRNA^{ile-ala} were found.

These ITS sequences were submitted to sequence analysis, and compared with the other spacers already sequenced.

The nucleotide sequence of the tRNA^{glu} spacer from the sample to be identified differed in 6 base pairs out of 403 (98,5% homologies) when compared to the consensus nucleotide sequence of the tRNA^{glu} spacer from the type-strain for S. *marcescens* represented by SEQ ID NO 2.

The nucleotide sequence of the tRNA^{ile-ala} spacer from this sample differed in 18 base pairs out of 471 (96,2% homologies) when compared to the consensus nucleotide sequence of the tRNA^{ile-ala} spacer of *S. marcescens* represented by SEQ ID NO 4.

In view of the high degree of homology, it could be inferred that the sample contained S. *marcescens.*

**Table 4**

| **SEQ ID NO** | **Sequences** |
|---|---|
| 1 | Cctaaagatattgmttcgagtggcgtgctcacacagattgtctgatgaaaaagtaacgagcaaaagcgtcataaaagtacggtgtcgtgtccccttcgtctagaggcctaggacaccgccctttcacggcggtaacaggggttcgaatcccta ggggacgccaagcttccgacccacccggtgaaagcggcggtcttcagtatctgacgatacaccatatcttaaagatgactttcgagtcatgtttaagatattgctctttaacaatctggaacaagctgaaaattgaaacatgacggctgaaattyaty cctccgtagatgtayyggratraagagtaacctgtcatagagtctctcaaatgtttgcarckygaacgrtggaaacatcttcgggttgtga |
| 2 | Cctaaagatattagttcgagtggcgtgctcacacagattgtctgatgaaaaagtaacgagcaaaagcgtcataaaagtacggtgtcgtgtccccttcgtctagaggcctaggacaccgcccttttcacggcggtaacaggggttcgaatcccctag gggacgccaagcttccgacccacccggtgaaagcggcggtctcaagtatctgacgatacaccatatcttaaagatgacttccgagtcatgtttaagatattgctctttaacaatctggaacaagctgaaaattgaaacatgacggytgaaatttatcc ctccgtagacgtattgggatraagagtaacctgtcatagagtctctcaaatgtttgcagcgcgaacgatggaaacatcttcgggttgtga |
| 3 | Cctaaagatattgmttcgagtggcgtgctcacacagattgtctgatnaraaagtaacgagcagaaatacctttataggcttgtagctcaggtggttagagcgcacccctgataagggtgaggtcggtggttcaagtccactcaggcctaccacttc tcggaagtggaaaaggtactgcgcgtgactgtatggggctatagctcagctgggagagcgcctgccttgcacgcaggaggtcagcggttcgatcccgcttagctccaccatatagtccggtatttcactacttcagagtatattggcaacagtatg ctgcgaagtattttgctctttaacaatctggaacaagctgaaaattgaaacatgacggctgaaattyatycctccgtagawgtaytgggataaagagtaacctgtcatagagtctctcaaatgtttgcaacttgaaygrtggaaacatcttcgggttgt ga |
| 4 | Cctaaagatattagttcgagtggcgtgctcacacagattgtctgatagaaagtaacgagcagaaatacctttataggcttgtagctcaggtggttagagcgcacccctgataagggtgaggtcggtggttcaagtccactcaggcctaccacttctc ggaagtggaaaaggtactgcgcgtgactgtatggggctatagctcagctgggagagcgcctgccttgcacgcaggaggtcagcggttcgatcccgcttagctccaccatatagtccggtatttaatacttcagagtatattggcgacagtatgct gcgaagtatttgctctttaacaatctggaacaagctgaaaattgaaacatgacggctgaaatttgtccctccgtagacgtattgggatgaagagtaacctgtcatagagtctctcaaatgtttgcagcgcgaacgatggaaacatcttcgggttgtga |
| 5 | Cctaaagatattagttcgcgtggcgtgctcacacagattgtctgatagaaagtaacgagcagaaatacctttataggcttgtagctcaggtggttagagcgcacccctgataagggtgaggtcggtggttcaagtccactcaggcctaccacttctt gtaagtggaaaaggtactgcacgtgactgtatggggctatagctcagctgggagagcgcctgccttgcacgcaggaggtcagcggttcgayccgcttagctccaccatatagtccggtatttcactacttcagagtatattggcaacagtatgct gcgaagtatttgctctttaacaatctggaacaagctgaaaattgaaacatgacggctgaaattcattcctccgtagatgtaccggaatgaagartaacctgtcatagagtctctcaaatgtttgcaacttraacggtggaaacatcttcgggttgtga |
| 6 | Cctaatgatattgattgcgtgaagtgctcacacagattgtctgatagaaaagtartgagcaaaagcgctacctgttgatgtaatgagtctctgactcatgctgatacgaaccgattaagacgtgagtctaatcggaatttcgtgtccccatcgtctaga ggcctaggacactgccctttcacggctgtaacaggggttcgaatccccttggggacgccattccgataatgagtgaaagacattatcaccggtgcttgctgcrccgaaaacatcttaaagatgactttaacgagtcgtgtttaagatattgctctttaa caatctggaacaagctgaaaattgaaacatgacagctgaaacttactcctccgtagaagtaytggagtaaggattaccctgtcatagagtctctcaaatgtagcagcrygaragtggaaacacttttgggttgtga |
| 7 | Cctaatgatattgattgcgtgaagtgctcacacagattgtctgatgaaatgaaagagagcataaggcttaatcttcgcggttaagtcttaaagcaccttgtcgggtctgtagctcaggtggttagagcgcacccctgataagggtgaggtcggtggt tcaagtccactcagacccaccaattctctcctatcctgcgttgtcgccctggttgtttacgtgagtaaacgtcgcaggacatcgctttgacgagaagcgaattggcgaaggtagtagataaggtgtgaaaaatgcttttctgatatggggctatagctc agctgggagagcgcctgccttgcacgcaggaggtcagcggttcgatcccgcttagctccaccatatagtcctgtatttcaatacttcagagtgtactggcaacagtatgctgcgaagtattttgctctttaacaatctggaacaagctgaaaattgaa acatgacagctgaaacttactcctccgtagaagtattggagtaaggagtaccctgtcatagagtctctcaaatgtagcagcatgaaagtggaaacacttttgggttgtga |
| 8 | Cctaaagatatgcagttgagtgcagtgtccacacagattgtctgatgaaaagtaatgagcaaagcgctacctgttgatgtaatgagtcttctggactcatgctgatacgagaaacggttaaaccctgggtttaatcggattttgtgtccccatcgtcta gaggcctaggacactgccctttcacggctgtaacaggggttcgaatcccccttggggacgccatccgataatgagtgaaagacattatcaccggtcattcgtgactgattatcttaaagatgactcttgcgagtcgtgtttaagatattgctctttaaca atctggaacaagctgaaaattgaaacatgacagctgaaacttatccctccgtagaagtattggggtaaggactaacctgtcatagagtctctcaaataaacagcacgacagtgtctctgtttacagaaacacccttcgggttgtga |
| 9 | Cctaaagatatgcagttaagtgcagtgtccacacagattgtctgatgaaaagtaatgagcaaagcgctacctgttgatgtaatgagtcttctggactcatgctgatacgaaccgattaagaagttttatttaatcggattttcgtgtccccatcgtctaga ggcctaggacactgccctttcacggctgtaacaggggttcgaatccccttggggacgccatccgataatgagtgaaagacattatcaccggtcattcgtgactgattatcttaaagatgactcttgcgagtcgtgtttaagatattgctctttaacaatc tggaacaagctgaaaattgaaacatgacagctgaaacttatccctccgtagaagtattggggtaaggattaacctgtcatagagtctctcaaataaacagcacgacagtgtctctgtttacagaaacaccttcgggttgtga |
| 10 | Cctaaagatatgcagttgagtgcagtgtccacacagattgtctgatgaaaaagtaacgagcagaaatacctttataggcttgtagctcaggtggttagagcgcacccctgataagggtgaggtcggtggttcaagtccactcaggcctaccaaatt |
| | cgcacttgtgctgcgttgcacgcgatacgtttacaccgtaaacgtccagtgagcatgccttgcctgagcgagtggtaaataaaggtttctgcaagtgactgtatggggctatagctcagctgggagagcgcctgccttgcacgcaggaggtcagc ggttcgatcccgcttagctccaccatatccatatggtccttgataatacttcagagtgtactggcaacagtatgctgcgaagtattttgctctttaacaatctggaacaagctgaaaattgaaacatgacagctgaaacttatccctccgtagaactattg gggtaaggattaacctgtcatagagtctctcaaataaacagcacgacagtgttctctgtttacagaaacaccttcgggttgtga |
| 11 | Cctaaagatatgcagttgagtgcagtgtccacacagattgtctgatgaaaaagtaacgagcagaaataccttttataggcttgtagctcaggtggttagagcgcacccctgataagggtgaggtcggtggttcaagtccactcaggcctaccaaatt cgcacttatgctgcgttatgcgcgcggtcgtttacccaaaagtaaacttcccttccgcatgccttgcctaagcacgaattaacagggtttccaacaggaaagactgttattcacgcgagtggtaaataaaggtttctgcaagtgactgtatggggctat agctcagctgggagagcgcctgccttgcacgcaggaggtcagcggttcgatcccgcttagctccaccatatccatatggtccttgataatacttcagagtgtactggcaacagtatgctgcgaagtattttgctctttaacaatctggaacaagctga aaattgaaacatgacagctgaaacttatccctccgtagawstattggggtaaggattaacctgtcatagagtctctcaaataaacagcacgacagtgttctctgtttacagaaacaccttcgggttgtga |
| 12 | Ctctttaacaatcaggaacaagctgaaaa |
| 13 | Attttgctctttaacaatctggaacaagctgaaaattg |
| 14 | Gacccacccggtgaaagcggcggtct |
| 15 | Acagtatgctgctgcgaagtattttgctctttaacaatc |
| 16 | Tctggaacaagctgaaaattgaaacatgacggctgaaat |
| 17 | Gctgcgaagtattttgctctttaac |
| 18 | Ctgcgaagtattttgctctttaac |
| 19 | Tgcgaagtattttgctctttaac |
| 20 | Tgcgaagtattttgctcttt |
| 21 | Cgaagtattttgctctttaacaatctggaacaa |
| 22 | Cgaagtattttgctctttaacaatctggaac |
| 23 | Cgaagtattttgctctttaacaatctggaacaagc |
| 24 | Acagtatgctgcgaagtattt |
| 25 | Acagtatgctgcgaagtat |
| 26 | Ttgctctttaacaatctggaacaa |
| 27 | Tgaaacatgacggctgaaatttatcc |
| 28 | Catgacggctgaa |
| 29 | Catgacggctgaaatttgtccctccg |
| 30 | Agtatctgacgatacaccatatcttaaagatgac |
| 31 | Agtatctgacgatacaccatatcttaaag |
| 32 | Agtatctgacgatacaccatatc |
| 33 | Ggaacaagctgaaaattgaaacatgac |
| 34 | Cattcctccgtagatgtaccggaatgaaga |
| 35 | Cattcctccgtagatgtaccggaatg |
| 36 | Tctggaacaagctgaaaattgaaacatgacggctgaaat |
| 37 | Tctggaacaagctgaaaattgaaacatgacggctg |
| 38 | Tctggaacaagctgaaaattgaaacatgac |
| 39 | Tggaacaagctgaaaattgaaacatg |
| 40 | Ctgaaaattgaaacatgacggctgaa |
| 41 | Ctgaaagttgaaacatgacggctgaa |
| 42 | Aaattgaaacatgacggctgaaattt |
| 43 | Aaattgaaacatgacggctgaaa |
| 44 | Gaaaattgaaacatgacggctga |
| 45 | Gaaagttgaaacatgacggctga |
| 46 | Aattgaaacatgacggctgaaa |
| 47 | Tctttaacaatctggaacaagctgaaaattg |
| 48 | Tgctctttaacaatctggaacaagctgaa |
| 49 | Gaaaattgaaacatgacggctgaaattt |
| 50 | Agcttccgacccacccggtgaaagcggcggtctymagtatctgacgatacaccatatcttaaagatgacttycgagtcatgtttaag |
| 51 | Aaagcgtcataaaagtacggtgtcgt |
| 52 | Cgagtcatgtttaag |
| 53 | Tacttcagagtatattggcracagtatgctgcgaagtattttgctctttaacaatctggaacaagctgaaaattgaaacatgacggctgaaatt |
| 54 | Tacttcagagtatattggc |
| 55 | Catagagtctctcaaatgtttgca |
| 56 | Gtggcgtgctcacacagat |
| 57 | Acagtatgctgcgaagtattttgctcttt |
| 58 | Acaccgcccgtcacaccayg |
| 59 | Astgccarggcatccacc |

## Claims

1. A set of two polynucleotide probes, each probe comprising 5 to 50 nucleotides, said probes hybridizing specifically to a nucleic acid selected from the group consisting of SEQ ID NO 1 to 11, their RNA form wherein T is replaced by U, the complementary form thereof, wherein there are no more than 25 nucleotides between said probes, for the detection and/or identification of *serratia* species.

2. A set of at least two polynucleotide probes according to claim 1, wherein said probes are selected from the group consisting of SEQ ID NO 12 to 57.

3. A set of three polynucleotide probes, each probe comprising 5 to 50 nucleotides, said probes hybridizing specifically to a nucleic acid selected from the group consisting of SEQ ID NO 1 to 11, their RNA form wherein T is replaced by U, the complementary form thereof, wherein there are no more than 25 nucleotides between said probes, for the detection and/or identification of *Serratia* species.

4. A composition comprising at least one set of polynucleotide probes according to any one of claims 1 to 3.

5. A method for detecting or identifying *Serratia* species using at least one set of polynucleotide probes according to any of claims 1 to 3.

6. A method according to claim 5 for detection and/or identification of *Serratia* species in a sample comprising the steps of:
(i) optionally releasing, isolating and/or concentrating the polynucleic acids in the sample;
(ii) optionally amplifying the 16S-23S rRNA spacer region(s), or the target sequence(s) which comprise(s) any nucleic acid molecule(s) according to anyone of claims 1 to 3, with at least one suitable primer pair;
(iii) contacting the polynucleic acids with at least one set of polynucleotide probes according to any one of claims 1-3 that hybridize to the target sequence(s) adjacent to each other with less than 25 nucleotides in between,
(iv) detecting the hybrids formed, and
(v) interpreting the signal(s) obtained and inferring the presence of *Serratia* species and/or identifying the *Serratia* species in the sample.

7. A method according to claim 5 or 6 wherein the two polynucleotide probes consist of any combination of polynucleotides of SEQ ID NO 12 to 49.

8. A kit for detection and/or identification of *Serratia* species comprising the following components:
- a set of polynucleotide probes described in any one of claims 1 to 3,
- a hybridization buffer, or components necessary for producing said buffer.

## Patentansprüche

1. Satz von zwei Polynukleotidsonden, wobei jede Sonde aus 5 bis 50 Nukleotiden besteht, wobei die Sonden spezifisch zu einer Nukleinsäure hybridisieren, ausgewählt aus der Gruppe bestehend aus SEQ ID NO 1 bis 11, ihrer RNA-Form, wobei T durch U ersetzt ist, der komplementären Form davon, wobei nicht mehr als 25 Nukleotide zwischen den Sonden vorhanden sind, zum Nachweis und/oder zur Identifizierung von *Serratia-*Spezies.

2. Satz von mindestens zwei Polynukleotidsonden nach Anspruch 1, wobei die Sonden ausgewählt sind aus der Gruppe bestehend aus SEQ ID NO 12 bis 57

3. Satz von drei Polynukleotidsonden, wobei jede Sonde aus 5 bis 50 Nukleotiden besteht, wobei die Sonden spezifisch zu einer Nukleinsäure hybridisieren, ausgewählt aus der Gruppe bestehend aus SEQ ID NO 1 bis 11, ihrer RNA-Form, wobei T durch U ersetzt ist, der komplementären Form davon, wobei nicht mehr als 25 Nukleotide zwischen den Sonden vorhanden sind, zum Nachweis und/oder zur Identifizierung von *Serratia-*Spezies.

4. Zusammensetzung, umfassend mindestens einen Satz von Polynukleotidsonden nach einem der Ansprüche 1 bis 3.

5. Verfahren zum Nachweis oder zur Identifizierung von *Serratia*-Spezies unter Verwendung von mindestens einem Satz Polynukleotidsonden nach einem der Ansprüche 1 bis 3.

6. Verfahren nach Anspruch 5 zum Nachweis und/oder zur Identifizierung von Serratia-Spezies in einer Probe, umfassend die folgenden Schritte:
(i) optional Freisetzen, Isolieren und/oder Konzentrieren der Polynukleinsäuren in der Probe;
(ii) optional Amplifizieren des/der 16S-23S rRNA-Abstandsbereichs/Abstandsbereiche oder des/der Zielsequenz(en), die jedes/alle Nukleinsäuremolekül(e) nach einem der Ansprüche 1 bis 3 umfasst/umfassen, mit mindestens einem geeigneten Primerpaar;
(iii) In-Kontakt-Bringen der Polynukleinsäuren mit mindestens einem Satz von Polynukleotidsonden nach einem der Ansprüche 1 - 3, die zu der/den Zielsequenz(en) hybridisieren, die einander mit weniger als 25 Nukleotiden dazwischen benachbart sind;
(iv) Nachweisen der gebildeten Hybride; und
(v) Interpretieren des erhaltenen Signals/der erhaltenen Signale und Ableiten der Anwesenheit von *Serratia*-Spezies und/oder Identifizieren von Serratia-Spezies in der Probe.

7. Verfahren nach Anspruch 5 oder 6, wobei die zwei Polynukleotidsonden aus jeder Kombination von Polynukleotiden von SEQ ID NO 12 bis 49 bestehen.

8. Kit zum Nachweis und/oder zur Identifizierung von *Serratia*-Spezies, umfassend die folgenden Komponenten:
- einen Satz von Polynukleotidsonden, beschrieben in einem der Ansprüche 1 bis 3,
- einen Hybridisierungspuffer oder Komponenten, die erforderlich sind, um den Puffer zu erzeugen.

## Revendications

1. Ensemble de deux sondes polynucléotidiques, chaque sonde comprenant de 5 à 50 nucléotides, lesdites sondes s'hybridant spécifiquement avec un acide nucléique choisi dans le groupe constitué des SEQ ID N° 1 à 11, de leur forme ARN dans laquelle T est remplacé par U, de la forme complémentaire de celle-ci, dans lequel il n'y a pas plus de 25 nucléotides entre lesdites sondes, pour la détection et/ou l'identification de l'espèce *Serratia.*

2. Ensemble d'au moins deux sondes polynucléotidiques selon la revendication 1, dans lequel lesdites sondes sont choisies dans le groupe constitué des SEQ ID N° 12 à 57.

3. Ensemble de trois sondes polynucléotidiques, chaque sonde comprenant de 5 à 50 nucléotides, lesdites sondes s'hybridant spécifiquement avec un acide nucléique choisi dans le groupe constitué des SEQ ID N° 1 à 11, de leur forme ARN dans laquelle T est remplacé par U, de la forme complémentaire de celle-ci, dans lequel il n'y a pas plus de 25 nucléotides entre lesdites sondes, pour la détection et/ou l'identification de l'espèce *Serratia.*

4. Composition comprenant au moins un ensemble de sondes polynucléotidiques selon l'une quelconque des revendications 1 à 3.

5. Procédé pour la détection ou l'identification de l'espèce *Serratia* au moyen d'au moins un ensemble de sondes polynucléotidiques selon l'une quelconque des revendications 1 à 3.

6. Procédé selon la revendication 5 pour la détection et/ou l'identification de l'espèce *Serratia* dans un échantillon, comprenant les étapes consistant à :
(i) éventuellement libérer, isoler et/ou concentrer les acides polynucléiques dans l'échantillon ;
(ii) éventuellement amplifier la ou les régions espaceur d'ARNr 16S-23S, ou la ou les séquences cibles qui comprennent éventuellement une ou plusieurs molécules d'acide nucléique selon l'une quelconque des revendications 1 à 3, avec au moins une paire d'amorces appropriées ;
(iii) mettre les acides polynucléiques au contact d'au moins un ensemble de sondes polynucléotidiques selon l'une quelconque des revendications 1 à 3 qui s'hybrident avec la ou les séquences cibles adjacentes les unes aux autres avec moins de 25 nucléotides entre elles,
(iv) détecter les hybrides formés, et
(v) interpréter le ou les signaux obtenus et en déduire la présence de l'espèce *Serratia* et/ou identifier l'espèce *Serratia* dans l'échantillon.

7. Procédé selon la revendication 5 ou 6, dans lequel les deux sondes polynucléotidiques sont constituées d'une quelconque combinaison des polynucléotides des SEQ ID N° 12 à 49.

8. Trousse de détection et/ou d'identification de l'espèce Serratia, comprenant les éléments suivants :
- un ensemble des sondes polynucléotidiques décrites dans l'une quelconque des revendications 1 à 3,
- un tampon d'hybridation, ou les éléments nécessaires à la production dudit tampon.
